(19) Europäisches Patentamt
European Patent Office
Office européen des brevets

(11) **EP 3 266 520 A1**

(12) **EUROPEAN PATENT APPLICATION**
published in accordance with Art. 153(4) EPC

(43) Date of publication:
**10.01.2018 Bulletin 2018/02**

(21) Application number: **16758956.3**

(22) Date of filing: **02.03.2016**

(51) Int Cl.:
*B01J 23/887* [(2006.01)]        *B01J 35/08* [(2006.01)]
*B01J 37/02* [(2006.01)]        *B01J 37/08* [(2006.01)]
*C07C 5/48* [(2006.01)]        *C07C 11/167* [(2006.01)]
*C07B 61/00* [(2006.01)]

(86) International application number:
**PCT/JP2016/056414**

(87) International publication number:
**WO 2016/140263 (09.09.2016 Gazette 2016/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 03.03.2015   JP 2015041831
03.03.2015   JP 2015041832
03.03.2015   JP 2015041833

(71) Applicant: **Nippon Kayaku Kabushiki Kaisha
Tokyo 100-0005 (JP)**

(72) Inventors:
• **NAKAZAWA Yuuta
Sanyoonoda-shi
Yamaguchi 757-0002 (JP)**

• **OKUMURA Shigeki
Sanyoonoda-shi
Yamaguchi 757-0002 (JP)**
• **MOTOMURA Hiroki
Sanyoonoda-shi
Yamaguchi 757-0002 (JP)**
• **NISHIZAWA Bungo
Sanyoonoda-shi
Yamaguchi 757-0002 (JP)**
• **NAKAYAMA Koji
Sanyoonoda-shi
Yamaguchi 757-0002 (JP)**

(74) Representative: **Gille Hrabal
Brucknerstrasse 20
40593 Düsseldorf (DE)**

(54) **CONJUGATED-DIOLEFIN-PRODUCING CATALYST, AND PRODUCTION METHOD THEREFOR**

(57)    Provided are a catalyst which, in a reaction for producing a conjugated diolefin by catalytic oxidative dehydrogenation from a mixed gas including a monoolefin having 4 or more carbon atoms and molecular oxygen, may suppress the production of a coke-like substance and improve the long-term stability of the reaction; and a method for producing the same. Disclosed is a composite metal oxide catalyst for producing a conjugated diolefin by a gas phase catalytic oxidative dehydrogenation reaction from a mixed gas including a monoolefin having 4 or more carbon atoms and molecular oxygen, the composite metal oxide catalyst having a solid acidity of 35 to 172 $\mu$mol/g.

**EP 3 266 520 A1**

**Description**

TECHNICAL FIELD

The present invention relates to a composite metal oxide catalyst (hereinafter, also described as catalyst) for producing a conjugated diolefin by a catalytic oxidative dehydrogenation reaction from a mixed gas including a monoolefin having 4 or more carbon atoms and molecular oxygen, and a method for producing the same.

BACKGROUND ART

Butadiene, which is a raw material of synthetic rubber or the like, has been conventionally produced industrially by thermal cracking and extraction of a naphtha fraction; however, in recent years, there are concerns that the supply to the market in the future may become unstable, and there is a demand for a new method for producing butadiene. Thus, attention has been paid to a method of oxidatively dehydrogenating n-butene in the presence of a catalyst from a mixed gas including n-butene and molecular oxygen. However, there are concerns that a coke-like substance originating from a reaction product and/or reaction by-products is precipitated in or adheres to the catalyst surface, inert materials, the interior of a reaction tube, or the interior of the subsequent process facilities, various problems such as inhibition of the circulation of the reaction gas in an industrial plant, blocking of the reaction tube, shutdown of the plant resulting therefrom, and a decrease in the yield, may be brought about. For the purpose of avoiding the problems described above, in an industrial plant, generally, the reaction is stopped before blocking occurs, and a regeneration treatment of combusting and removing the coke-like substance by means of temperature increase of the reaction bath temperature or the like is carried out. However, since stopping a reaction that is in steady operation and then performing a regeneration treatment leads to the deterioration of economic efficiency in an industrial plant, it is preferable that the production of the coke-like substance is suppressed as far as possible. Thus, it is known to make improvements in the catalyst composition, reaction conditions, and the like as described below, for the purpose of suppressing the generation of the coke-like substance.

Regarding patent literatures related to the catalyst composition, Patent Literature 1 discloses a catalyst composition that suppresses side-production of a coke-like substance and enables stable continuation of the reaction, and Patent Literature 3 discloses a catalyst composition and the acidity of the catalyst that give high conjugated diolefin yield. Furthermore, the X-ray diffraction peak ratio of a catalyst that may suppress the generation of a coke-like substance is defined in Patent Literature 4. Regarding patent literatures related to the reaction conditions, reaction conditions that prevent accumulation of a coke-like substance on a catalyst are defined in Patent Literature 2. In regard to all of these patent literatures, when the economic efficiency in the industrial plant, that is, the amount of precipitation of a coke-like substance with respect to the duration of the reaction, the frequency of regeneration, and the like are taken into consideration, it is inappropriate to say that the production of coke-like substances has been sufficiently suppressed. Thus, it has been desired that a catalyst composition that may further suppress the production of a coke-like substance and improve the long-term stability of the reaction, and reaction conditions for the catalyst, will be suggested.

From the viewpoint of the economic efficiency in an industrial plant, it is also definitely important that a conjugated diolefin, which is a target product, is obtained with high yield. A low yield of a conjugated diolefin implies that the yield of by-products is relatively high; however, in this case, in order to obtain a conjugated diolefin of high purity as a final product in an industrial plant, a purification system with superior performance is needed, and there are concerns about the facilities cost of such a system being increased.

Furthermore, from the viewpoint of the long-term stability of the reaction in an industrial plant, it is also important that the mechanical strength of the catalyst itself is high. If the catalyst has not sufficient mechanical strength, problems such as damage of the catalyst caused by the production of the coke-like substance, and/or damage of the catalyst caused by combustion gas in the regeneration treatment, and/or degradation of the catalyst, may be considered. Furthermore, there are concerns that these problems may lead to other problems such as accumulation of damaged catalyst in the reactor, an increase in the pressure loss, undesirable reactions caused by the catalyst that has locally accumulated in the reactor, and incorporation of the accumulated catalyst in the purification system in the subsequent stages.

CITATION LIST

PATENT LITERATURE

Patent Literature 1: JP 2013-100244 A
Patent Literature 2: JP 2011-148765 A

Patent Literature 3: JP 2013-146655 A
Patent Literature 4: JP 2013-202459 A

SUMMARY OF INVENTION

TECHNICAL PROBLEM

An object of the present invention is to provide a catalyst that may suppress the production of a coke-like substance and improve the long-term stability of the reaction, in a reaction for a production a conjugated diolefin from a mixed gas including a monoolefin having 4 or more carbon atoms and molecular oxygen by a catalytic oxidative dehydrogenation reaction, and to provide a method for producing the catalyst.

SOLUTION TO PROBLEM

The inventors of the present invention conducted a thorough investigation in order to solve the problems described above, and as a result, the inventors found that when the acidity per unit weight of the catalyst satisfies a particular range, production of the coke-like substance may be significantly suppressed, and the problems described above may be solved. Thus, the inventors completed the present invention.
The present invention has the feature of the following items (1) to (9) singly or in combination. That is, the present invention may include the following embodiments:

(1) a composite metal oxide catalyst for producing a conjugated diolefin by a gas phase catalytic oxidative dehydrogenation reaction from a mixed gas including a monoolefin having 4 or more carbon atoms and molecular oxygen, the composite metal oxide catalyst having a solid acidity of 35 to 172 $\mu$mol/g,
(2) the composite metal oxide catalyst according to (1), including molybdenum (Mo), bismuth (Bi), an alkali metal (AM), and if necessary, an alkaline earth metal (AEM), wherein the molar ratio of the alkali metal and the alkaline earth metal with respect to bismuth, Bi/(AM + AEM), is from 0.6 to 11.0,
(3) the composite metal oxide catalyst according to (1) or (2), wherein the composite metal oxide catalyst satisfies the following composition formula:

$$Mo_{12}Bi_aFe_bCo_cNi_dX_eY_fZ_gO_h$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium, and barium; Z represents at least one element selected from lanthanum, cerium, praseodymium, neodymium, samarium, europium, antimony, tungsten, lead, zinc, cerium, and thallium; O represents oxygen; a, b, c, d, e, and f represent atomic ratios of bismuth, iron, cobalt, nickel, the alkali metal, the alkaline earth metal, and Z, respectively, with respect to molybdenum 12; and h represents a value that satisfies oxidation states of other elements in ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$,
(4) a method for producing the composite metal oxide catalyst for conjugated diolefin production according to (2), the method including steps of:

preparing a slurry including a compound containing molybdenum; a compound containing bismuth; a compound containing at least one alkali metal; and if necessary, a compound containing at least one alkaline earth metal, and drying the slurry to obtain a dry powder; and
calcining the dry powder at a temperature of from 200°C to 600°C,

(5) a method for producing the composite metal oxide catalyst for conjugated diolefin production according to (3), the method including steps of:

preparing a slurry including a compound containing molybdenum; a compound containing bismuth; a compound containing iron; a compound containing cobalt; a compound containing nickel; a compound containing at least one alkali metal element selected from lithium, sodium, potassium, rubidium, and cesium; a compound containing at least one alkaline earth metal element selected from magnesium, calcium, strontium, and barium; and a compound containing at least one element Z selected from lanthanum, cerium, praseodymium, neodymium, samarium, europium, antimony, tungsten, lead, zinc, cerium, and thallium,
provided that a, b, c, d, e, and f represent the atomic ratios of bismuth, iron, cobalt, nickel, the alkali metal, the alkaline earth metal, and Z, respectively, with respect to molybdenum 12, and in the ranges of 0.3 < a < 3.5, 0.6 < b < 3.4, 5 < c < 8, 0 < d < 3, 0 < e < 0.5, 0 ≤ f ≤ 4.0, 0 ≤ g ≤ 2.0;
drying the slurry to obtain a dry powder; and
calcining the dry powder at a temperature of from 200°C to 600°C,

(6) a supported catalyst for conjugated diolefin production, the supported catalyst having the composite metal oxide catalyst according to any one of (1) to (3) supported on a support,

(7) the supported catalyst according to (6), wherein the support is a spherical support,

(8) the supported catalyst according to (6) or (7), wherein the average particle size is from 3.0 mm to 10.0 mm, and the support ratio of the composite metal oxide catalyst is from 20% by weight to 80 weight, and

(9) a method for producing the supported catalyst for conjugated diolefin production according to any one of (6) to (8), the method including a molding step of coating a support with a composite metal oxide catalyst together with a binder.

## ADVANTAGEOUS EFFECTS OF INVENTION

A catalyst which may suppress the production of a coke-like substance and improve the long-term stability of the reaction, in a reaction for producing a conjugated diolefin from a mixed gas including a monoolefin having 4 or more carbon atoms and molecular oxygen by catalytic oxidative dehydrogenation, and a method for producing the catalyst are provided.

## DESCRIPTION OF EMBODIMENTS

Manifestation of the solid acid point is generally known to be attributed to the generation of defects in the crystal structure due to the difference in the existence value of metal elements or the degree of oxidation between adjacent elements in a composite metal oxide catalyst, or to be attributed to the characteristics of the metal oxide itself. However, in the present composite metal oxide catalyst, the solid acidity is 35 to 172 $\mu$mol/g, preferably 59 to 138 $\mu$mol/g, and more preferably 79 to 117 $\mu$mol/g.

In order to regulate the amount of solid acid points, that is, the solid acidity, it is generally well known that the ratio of constituent atoms in a composite metal oxide catalyst is regulated, that alkali metal elements and/or alkaline earth metal elements are mixed by varying the type or the amount, that the calcination temperature is regulated, or that the catalyst surface is treated with an organic acid or the like. In regard to the present composite metal oxide catalyst, any means may be employed as long as the scope described above is satisfied.

Next, the method for measuring the solid acidity according to the present invention will be explained.

For the measurement of the solid acidity, a temperature-programmed ammonia desorption method, which is one of generally performed methods, is used. The temperature-programmed ammonia desorption method is a method of using ammonia molecule having basicity as a probe molecule, adsorbing the ammonia molecules to the acid points present on the surface of a composite metal oxide catalyst, and subsequently measuring the amount of the solid acid molecules that make pairs with the ammonia molecules released by raising temperature.

In regard to the question of why a composite metal oxide catalyst having a solid acidity that satisfies a particular range according to the present invention may suppress the production of the coke-like substance in a reaction for producing a conjugated diolefin from a mixed gas including a monoolefin having 4 or more carbon atoms and molecular oxygen, the details are not clearly understood; however, the following may be considered as one of the causes. As a factor of the production of the coke-like substance, it may be considered that a conjugated diolefin as a main product and/or an acid or aldehyde having 2 to 4 carbon atoms as a side product, an oxidation product having a cyclic structure having 6 or more carbon atoms, or the like are chemically bonded, the products are caused to undergo a chain reaction, the molecular weight becomes excessively large to obtain a polymer form, and then a dehydrogenation reaction induced by heat or a catalyst proceeds, thereby a coke-like substance being produced. In that case, a solid acid point may be regarded as an activation point that promotes chemical bonding. The amount of solid acid points, that is, the solid acidity is necessary to a certain extent in order to implement an intended chemical reaction. However, in a case in which the solid acidity deviates from a particular range, it is speculated that the reaction rate of the side reaction is accelerated, and a chemical reaction is likely to proceed toward the production of a coke-like substance as described above.

There are no particular limitations on the technique for adjusting the solid acidity to a particular range according to the present invention, and the effects of the present invention may be obtained by using those generally used techniques singly or in combination. For example, it is desirable to adjust the composition of the composite metal oxide catalyst to be such that the molar ratio between bismuth (Bi) and an alkali metal (AM) together with an alkaline earth metal (AEM), Bi/(AM + AEM), with respect to 12 moles of molybdenum will be from 0.6 to 11.0, preferably 3.0 to 8.5, and more preferably 5.0 to 7.0.

It is preferable that the present catalyst satisfies the composition formula represented by Formula (1) :

$$Mo_{12}Bi_aFe_bCo_cNi_dX_eY_fZ_gO_h \qquad (1)$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium, and barium; Z represents at least one element selected from lanthanum, cerium, praseodymium, neodymium, samarium, europium, antimony, tungsten, lead, zinc, cerium, and thallium; O represents oxygen; a, b, c, d, e, and f represent the atomic ratios of bismuth, iron, cobalt, nickel, an alkali metal, an alkaline earth metal, and Z, respectively, with respect to molybdenum 12; and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$, h represents a value that satisfies the oxidation state of the other elements.

There are no particular limitations on the raw materials of the various metal elements for obtaining the present catalyst; however, nitrates, nitrites, sulfates, ammonium salts, organic acid salts, acetates, carbonates, subcarbonates, chlorides, inorganic acids, inorganic acid salts, heteropolyacids, heteropolyacid salts, hydroxides, oxides, metals, alloys, and the like, all of which include at least one of the various metal elements, and mixtures thereof may be used. Among these, preferred are nitrate raw materials.

The method for producing the present catalyst is not particularly limited; however, the production methods may be roughly classified into the following two types of production methods. For convenience, these methods will be referred to as Method (A) and Method (B) in this invention. Method (A) is a method of obtaining the active components of the catalyst as a powder, and then molding this powder, and Method (B) is a method of bringing a solution in which the active components of the catalyst are dissolved, into contact with a support that has been molded in advance, and thereby supporting the active components on the support. The details of Method (A) and Method (B) will be described below.

A method for catalyst production according to Method (A) will be described below. The order of the various processes is described below as a preferred example; however, there are no particular limitations on the sequence of the various processes, the number of processes, and combinations of the various processes for obtaining the final manufactured product of catalyst.

Step (A1) Production and drying

A mixed solution or slurry of raw material compounds is prepared, and the mixed solution or slurry is subjected to the processes of a precipitation method, a gelation method, a co-precipitation method, a hydrothermal synthesis method, or the like. Subsequently, a dried powder of the present invention is obtained by using a known drying method such as a dry spraying method, an evaporation drying method, a drum drying method, or a freeze-drying method. Here, for the mixed solution or the slurry, the solvent may be any of water, an organic solvent, or a mixed solution thereof, and there are also no limitations on the raw material concentration of the active components of the catalyst. Furthermore, there are no particular limitations on the liquid temperature of the mixed solution or the slurry, the conditions for production such as the atmosphere, and the drying conditions; however, it is definitely adequate to select the conditions from appropriate ranges in consideration of the performance, mechanical strength, and moldability of the catalyst finally obtained, or the production efficiency. Among these, a preferred method for the present invention is a method of forming a slurry of raw materials of the active components of the catalyst in a temperature range of 20°C to 90°C, introducing this slurry into a spray dryer, and regulating the hot air inlet temperature, the pressure inside the spray dryer, and the flow amount of the mixed solution or slurry, such that the dryer outlet temperature would be 70°C to 150°C, and the average particle size of the dried powder thus obtained would be 10 to 700 $\mu$m.

Step (A2) Preliminary calcination

The dried powder thus obtained is preliminarily calcined at 200°C to 600°C, and a preliminarily calcined powder of the present invention may be obtained. Here, also with regard to the conditions for the preliminary calcination, there are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions for the preliminary calcination from appropriate ranges in consideration of the performance, mechanical strength, and moldability of the catalyst finally obtained, or the production efficiency. Among these, a preferred method for the present invention is a method of performing preliminary calcination in a tunnel calcination furnace at a temperature in the range of 300°C to 600°C for 1 to 12 hours in an air atmosphere.

Step (A3) Molding

The preliminarily calcined powder obtained as described above may be directly used as a catalyst, but may also be used after molding. The shape of the molded article is not particularly limited and may be selected from a spherical shape, a cylindrical shape, a ring shape, and the like. However, the shape should be selected in consideration of the mechanical strength of the catalyst that is finally obtainable after a series of production processes, the reactor, the production efficiency for the preparation, and the like. The molding method is also not particularly limited; however, when the support, molding aids, strength enhancing agent, binder, and the like that will be described in the following paragraph are added to the preliminarily calcined powder and molded into a cylindrical shape or a ring shape, a tablet molding machine, an extrusion molding machine or the like is used, or when the mixture is molded into a spherical shape, a granulator or the like is used to obtain a molded article. Among these, a preferred method for the present invention is a method of coating an inert spherical support with the preliminarily calcined powder by a tumbling granulation method, and thus supporting and molding the catalyst.

Regarding the material for the support, known materials such as alumina, silica, titania, zirconia, niobia, silica-alumina, silicon carbide, a carbide, and mixtures thereof may be used. Furthermore, there are no particular limitations on the particle size, the water absorption rate and mechanical strength of the support, the degrees of crystallization of the various crystal phases, or the mixing ratio of the crystal phases, and it is definitely adequate to select the conditions for the support from appropriate ranges in consideration of the performance and moldability of the catalyst finally obtained, or the production efficiency. The mixing ratio of the support and the preliminarily calcined powder is calculated as a support ratio by the following formula, based on the feed weights of the various raw materials.

$$\text{Support ratio (wt\%)} = \text{(Weight of the preliminarily calcined powder used for molding)} / \{\text{(weight of the preliminarily calcined powder used for molding)} + \text{(weight of the support used for molding)}\} \times 100$$

Regarding the raw materials other than the preliminarily calcined powder used for molding, the preliminarily calcined powder, as well as any types of a molding aid such as crystalline cellulose; a strength enhancing agent such as ceramic whiskers; a binder such as an alcohol, a diol or a triol; and aqueous solutions thereof and the like may be used at any arbitrary mixing proportions to perform molding, and there are no particular limitations. It is also possible to introduce an element into the outermost surface of the catalyst in an embodiment that is different from Step (A1), by using a solution of the above-mentioned catalyst raw materials for this binder.

Step (A4) Main calcination

It is preferable that the preliminarily calcined powder or the molded article obtained as described above is subjected again to main calcination at 200°C to 600°C before being used in a reaction. Also, in connection with the main calcination, there are no particular limitations on the calcination time or the atmosphere during calcination, the technique for calcination is also not particularly limited and may be selected from a fluidized bed, a rotary kiln, a muffle furnace, a tunnel calcination furnace, and the like. It is definitely adequate to select the conditions for the main calcination from appropriate ranges in consideration of the performance and mechanical strength of the catalyst finally obtained, or the production efficiency. Among these, a preferred method for the present invention is a method of performing calcination in a tunnel calcination furnace at a temperature in the range of 300°C to 600°C for 1 to 12 hours in an air atmosphere.

Next, a method for catalyst production according to Method (B) will be described below. The various processes will be sequentially described in the following description; however, it should be noted that there are no particular limitations on the sequence of the various processes, the number of processes, and combinations of the various processes for obtaining the final manufactured product of catalyst.

Step (B1) Impregnation

A mixed solution or slurry into which the active components of the catalyst have been introduced is prepared, and a molded support or a catalyst obtained by Method (A) is impregnated with this solution or slurry. Thus, a molded article is obtained. Here, the technique for supporting the active components of the catalyst by impregnation is not particularly limited and may be selected from a dipping method, an incipient wetness method, an ion exchanging method, a pH swing method, and the like. As the solvent for the solution or the slurry, any of water, an organic solvent, or a mixed solution thereof may be used, and there are also no limitations on the raw material concentrations of the active components of the catalyst. Furthermore, there are also no particular limitations on the liquid temperature of the mixed solution or the slurry, the pressure applied to the liquid, and the atmosphere around the liquid. However, it is definitely adequate to select the conditions from appropriate ranges in consideration of the performance, mechanical strength and moldability of the catalyst finally obtained, or the production efficiency. The shapes of both the molded support and the catalyst obtained by Method (A) are not particularly limited and may be selected from a spherical shape, a cylindrical shape, a ring shape, a powdered form, and the like. There are also no particular limitations on the material, the particle size, the water absorption ratio, and the mechanical strength, with the scope of claims of the present patent application.

Step (B2) Drying

The molded article obtained as described above is subjected to a heat treatment at a temperature in the range of 20°C to 200°C using a known drying method such as an evaporation drying method, a drum drying method, or a freeze-drying method, and thus a catalyst dry molded product of the present invention is obtained. There are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions from appropriate ranges in consideration of the performance, mechanical strength and moldability of the catalyst finally obtained, or the production efficiency.

Step (B3) Main calcination

The molded product obtained as described above is subjected to a heat treatment at a temperature in the range of 200°C to 600°C using a known drying method such as an evaporation drying method, a drum drying method, or a freeze-drying method, and thus a catalyst of the present invention is obtained. Here, there are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions from appropriate ranges in consideration of the performance, mechanical strength and moldability of the catalyst finally obtained, or the production efficiency. Among these, a preferred method for the present invention is a method of performing calcination in a tunnel calcination furnace at a temperature in the range of 300°C to 600°C for 1 to 12 hours in an air atmosphere.

The catalyst obtained by the method described above is not particularly limited in view of the shape or the size; however, when the workability of packing of the reaction tube, the pressure loss in the reaction tube after packing, and the like are considered, the shape is preferably a spherical shape, the average particle size is preferably 3.0 to 10.0 mm, and the support ratio of the composite metal oxide catalyst is preferably 20% to 80% by weight.

It is preferable that the present catalyst has a certain mechanical strength at least before the initiation of the reaction, as will be described below. As described above, in a case in which a reaction for producing a conjugated diolefin from a monoolefin having 4 or more carbon atoms is carried out particularly in an industrial plant, the production of a coke-like substance and a regeneration treatment for combusting the coke-like substance are carried out in many occasions. Thus, if the catalyst has insufficient mechanical strength, damage of the catalyst caused by the production of a coke-like substance inside the catalyst, and damage of the catalyst and/or deterioration of the catalyst caused by the combustion gas in the regeneration treatment, may be considered. Also, there are concerns that the damaged catalyst accumulates within the reactor, and this may lead to various problems such as an increase in the pressure loss, undesirable reactions caused by the catalyst that has locally accumulated within the reactor, and/or a decrease in yield, and incorporation into the purification system in the subsequent stages. Here, the mechanical strength of a catalyst is affected by various factors in the production process, such as the type or amount of the various strength enhancing agents or binders added at the time of molding, or a combination thereof, the atomic ratio of the catalyst composition, the phase morphology of various crystal phases and proportions thereof, and the diameter, the geometric structure and the form of aggregation of secondary particles of the composite metal oxide catalyst formed in the production process or the drying process, and the mechanical strength is also closely related to the performance of the catalyst. That is, it may be said that the object of the present invention more particularly relates to the demand for a catalyst which simultaneously satisfies suppression of the production of the coke-like substance, a high yield of the conjugated diolefin, and high mechanical strength in a reaction for producing a conjugated diolefin from a monoolefin having 4 or more carbon atoms.

The degree of wear, which is an index representing the mechanical strength according to the present invention, is calculated by the following method. A tester for the degree of wear of tablets manufactured by Hayashi Rikagaku K.K. is used as an apparatus, and 50 g of a catalyst sample is treated at a speed of rotation of 25 rpm for a treatment time of 10 minutes. Subsequently, the portion that has worn out is sieved with a standard sieve having a mesh size of 1.70 mm, and the weight of the catalyst remaining on the sieve is measured. The degree of wear is calculated by the following formula. As the value of the degree of wear is smaller, superior mechanical strength is obtained. A preferred range of the degree of wear is, for example, 3% by weigh tor less, more preferably 1.5% by weight or less, and even more preferably 0.5% by weight or less.

$$\text{Degree of wear (wt\%)} = 100 \times [(\text{Weight of catalyst} - \text{weight of catalyst remaining on the sieve})/\text{weight of catalyst}]$$

The conditions for the reaction of producing a conjugated diolefin from a monoolefin having 4 or more carbon atoms by means of the present catalyst include the following: a mixed gas including 1% to 20% by weight of a monoolefin, 5% to 20% by weight of molecular oxygen, 0% to 60% by weight of water vapor, and 0% to 94% by weight of an inert gas, for example, nitrogen or carbon dioxide, is used, the reaction bath temperature is in the range of 200°C to 500°C, the reaction pressure is from normal pressure to an added pressure of 10 atmospheres, and the space velocity (GHSV) of the raw material gas with respect to the present catalyst molded article is in the range of 350 to 7,000 hr$^{-1}$. Regarding the form of the reaction, the reaction may be performed in a fixed bed, a movable bed, and a fluidized bed without any restrictions; however, a fixed bed is preferred.

The present catalyst may be used for a reaction by which a conjugated diolefin is produced from a mixed gas including a monoolefin having 4 or more carbon atoms and molecular oxygen by a catalytic oxidative dehydrogenation reaction. In a narrower sense, the present catalyst may be used for a reaction by which butadiene is produced from a mixed gas including n-butene and molecular oxygen by a catalytic oxidative dehydrogenation reaction.

n-Butene according to the present invention is intended to mean a single component gas selected from among 1-butene, trans-2-butene, cis-2-butene and isobutylene, or a mixed gas including at least one component, and more strictly, butadiene is intended to mean 1,3-butadiene.

The coke-like substance according to the present invention is produced from at least any one of the reaction raw materials, the reaction product, or the reaction by-products in the reaction for producing a conjugated diolefin, and the details of the chemical composition or the mechanism of generation are not clearly understood. However, the coke-like substance is regarded as a causative substance that causes various problems, particularly such as inhibition of the circulation of reaction gases in an industrial plant, blocking of reaction tubes, and shutdown of the reaction resulting therefrom, when the coke-like substance is precipitated in or adheres to an inert substance, inside the reaction tube, or inside the facilities in the subsequent processes. Furthermore, for the purpose of avoiding the problems described above, in industrial plants, generally reactions are stopped before blocking occurs, and a regeneration treatment of combusting and removing the coke-like substance by raising the reaction bath temperature, or the like. Regarding the mechanism for the production of the coke-like substance, for example, the following is assumed. That is, examples may include, but not limited to, that at the time of using a composite metal oxide catalyst including molybdenum, the coke-like substance is caused by polymerization of various olefins and condensation of high boiling point compounds, starting from the molybdenum compounds that have sublimed and precipitated in the reactor; the coke-like substance is caused by polymerization of various olefins and condensation of high boiling point compounds, starting from the abnormal acid-base points and radical production points inside the catalyst and the reactor; and the coke-like substance is caused by production of high boiling point compounds as a result of the Diels-Alder reaction of the conjugated diene and other olefin compounds, and by condensation at sites where the temperature is locally low inside the reactor. In addition to these, various mechanisms are known.

According to the present invention, there are obtained a catalyst that may suppress the production of a coke-like substance and improve the long-term stability of the reaction, in regard to a reaction by which a conjugated diolefin is produced from a mixed gas including a monoolefin having 4 or more carbon atoms and molecular oxygen by catalytic oxidative dehydrogenation; a method for producing the catalyst; and a method for producing a conjugated diolefin by using the catalyst described above.

EXAMPLES

Hereinafter, the present invention will be described in more detail by way of Examples; however, the present invention is not intended to be limited to the following Examples as long as the gist is maintained. In the following description, unless particularly stated otherwise, percentage (%) means mol%. Also, in the following description, the definitions of the n-butene conversion ratio, the butadiene yield, and TOS are as follows.

$$\text{n-Butene conversion ratio (mol\%)}$$

$$= \text{(Number of moles of reacted n-butene/number of moles}$$

$$\text{of supplied n-butene)} \times 100$$

$$\text{Butadiene yield (mol\%)}$$

$$= \text{(Number of moles of produced butadiene/number of}$$

$$\text{moles of supplied n-butene)} \times 100$$

TOS = Mixed gas circulation time (hours)

Example 1

(Production of catalyst 1)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 165 parts by weight of bismuth nitrate was dissolved in an aqueous solution of nitric acid prepared by adding 42 parts by weight of nitric acid (60% by weight) to 175 ml of pure water that had been heated to 60°C, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 1 of the present invention was obtained. The atomic ratio of Catalyst 1 calculated from the supplied raw materials was such that Mo : Bi : Fe : Co : Ni : Cs = 12 : 0.9 : 1.8 : 7.0 : 1.0 : 0.15, and the degree of wear was 0.41% by weight. The solid acidity of Catalyst 1 was 98 $\mu$mol/g.

Example 2

(Production of catalyst 4)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 165 parts by weight of bismuth nitrate was dissolved in an aqueous solution of nitric acid prepared by adding 42 parts by weight of nitric acid (60% by weight) to 175 ml of pure water that had been heated to 60°C, and the resulting solution was added to Mother Liquor 1. Furthermore, 45 parts by weight of calcium nitrate was dissolved in 50 parts by weight of pure water, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 4 of the present invention was obtained. The atomic ratio of Catalyst 4 calculated from the supplied raw materials was such that Mo : Bi : Fe : Co : Ni : Cs : Ca = 12 : 0.9 : 1.8 : 7.0 : 1.0 : 0.15 : 0.5, and the solid acidity of Catalyst 4 was 68 $\mu$mol/g.

Example 3

(Production of Catalyst 5)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 165 parts by weight of bismuth nitrate was dissolved in an aqueous solution of nitric acid prepared by adding 42 parts by weight of nitric acid (60% by weight) to 175 ml of pure water that had been heated to 60°C, and the resulting solution was added to Mother Liquor 1. Furthermore, 48 parts by weight of magnesium nitrate was dissolved in 50 parts by weight of pure water, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 5 of the present invention was obtained. The atomic ratio of Catalyst 5 calculated from the supplied raw materials was such that Mo : Bi : Fe : Co : Ni : Cs : Mg = 12 : 0.9 : 1.8 : 7.0 : 1.0 : 0.15 : 0.5, and the solid acidity of Catalyst 5 was 47 $\mu$mol/g.

Example 4

(Production of Catalyst 6)

800 parts by weight of ammonium heptamolybdate was completely dissolved in 3,000 parts by weight of pure water that had been heated to 80°C (Mother Liquor 1). Next, 6.6 parts by weight of cesium nitrate was dissolved in 75 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 165 parts by weight of bismuth nitrate was dissolved in an aqueous solution of nitric acid prepared by adding 42 parts by weight of nitric acid (60% by weight) to 175 ml of pure water that had been heated to 60°C, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 6 of the present invention was obtained. The atomic ratio of Catalyst 6 calculated from the supplied raw materials was such that Mo : Bi : Fe : Co : Ni : Cs = 12 : 0.9 : 1.8 : 7.0 : 1.0 : 0.09, and the solid acidity of Catalyst 6 was 49 $\mu$mol/g.

Example 5

(Production of Catalyst 7)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 238 parts by weight of bismuth nitrate was dissolved in an aqueous solution of nitric acid prepared by adding 61 parts by weight of nitric acid (60% by weight) to 252 ml of pure water that had been heated to 60°C, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 7 of the present invention was obtained. The atomic ratio of Catalyst 7 calculated from the supplied raw materials was such that Mo : Bi : Fe : Co : Ni : Cs = 12 : 1.3 : 1.8 : 7.0 : 1.0 : 0.15, and the solid acidity of Catalyst 7 was 39 $\mu$mol/g.

Example 6

(Production of Catalyst 8)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 275 parts by weight of bismuth nitrate was dissolved in an aqueous solution of nitric acid prepared by adding 70 parts by weight of nitric acid (60% by weight) to 291 ml of pure water that had been heated to 60°C, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 8 of the present invention was obtained. The atomic ratio of Catalyst 8 calculated from the supplied raw materials was such that Mo : Bi : Fe : Co : Ni : Cs = 12 : 1.5 : 1.8 : 7.0 : 1.0 : 0.15, and the solid acidity of Catalyst 8 was 42 $\mu$mol/g.

Comparative Example 1

(Production of Catalyst 2)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 311 parts by weight of bismuth nitrate was dissolved in an aqueous solution of nitric acid prepared by adding 79 parts by weight of nitric acid (60% by weight) to 330 ml of pure water that had been heated to 60°C, and the resulting solution was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 520°C and 5 hours, and Catalyst 2 for comparison was obtained. The atomic ratio of Catalyst 2 calculated from the supplied raw materials was such that Mo : Bi : Fe : Co : Ni : Cs = 12 : 1.7 : 1.8 : 7.0 : 1.0 : 0.15, and the degree of wear was 0.31% by weight. The solid acidity of Catalyst 2 was 173 $\mu$mol/g.

Comparative Example 2

(Production of Catalyst 3)

800 parts by weight of ammonium heptamolybdate was completely dissolved in 3,000 parts by weight of pure water that had been heated to 80°C (Mother Liquor 1). Next, 5.5 parts by weight of cesium nitrate was dissolved in 62 ml of pure water, and the resulting solution was added to Mother Liquor 1. Next, 229 parts by weight of ferric nitrate, 769 parts by weight of cobalt nitrate, and 110 parts by weight of nickel nitrate were dissolved in 587 ml of pure water that had been heated to 60°C, and the resulting solution was added to Mother Liquor 1. Subsequently, 183 parts by weight of bismuth nitrate was dissolved in an aqueous solution of nitric acid prepared by adding 47 parts by weight of nitric acid (60% by weight) to 194 ml of pure water that had been heated to 60°C, and the resulting solution was added to Mother Liquor 1. Furthermore, 28 parts by weight of calcium hydroxide and 97 parts by weight of magnesium nitrate were added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 560°C and 5 hours, and Catalyst 3 for comparison was obtained. The atomic ratio of Catalyst 3 calculated from the supplied raw materials was such that Mo : Bi : Fe : Co : Ni : Cs : Ca : Mg = 12 : 1 : 1.5 : 7.0 : 1.0 : 0.075 : 1 : 1, and the degree of wear was 0.48% by weight. The solid acidity of Catalyst 3 was 34 $\mu$mol/g.

Comparative Example 3

(Production of Catalyst 9)

800 parts by weight of ammonium heptamolybdate was completely dissolved in 3,000 parts by weight of pure water that had been heated to 80°C (Mother Liquor 1). Next, 13.2 parts by weight of cesium nitrate was dissolved in 150 ml of pure water, and the resulting solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the resulting solution was added to Mother Liquor 1. Subsequently, 110 parts by weight of bismuth nitrate was dissolved in an aqueous solution of nitric acid prepared by adding 28 parts by weight of nitric acid (60% by weight) to 117 ml of pure water that had been heated to 60°C, and the resulting solution was added to Mother Liquor 1. Furthermore, 97 parts by weight of magnesium nitrate was added to Mother Liquor 1. This Mother Liquor 1 was dried by a spray drying method, and the dried powder thus obtained was preliminarily calcined under the conditions of 440°C and 5 hours. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 500°C and 5 hours, and Catalyst 9 for comparison was obtained. The atomic ratio of Catalyst 9 calculated from the supplied raw materials was such that Mo : Bi : Fe : Co : Ni : Cs : Mg = 12 : 0.6 : 1.8 : 7.0 : 1.0 : 0.18 : 1. The solid acidity of Catalyst 9 was 30 $\mu$mol/g.

Test Example 1

(Coke precipitation reaction)

Catalyst 1 of the present invention thus obtained was reacted and evaluated by the following method. 53 ml of the catalyst was packed in a stainless steel reaction tube, and the catalyst was caused to react for a TOS of 290 hours using a mixed gas having a gas volume ratio of 1-butene : oxygen : nitrogen : water vapor = 1 : 1 : 7 : 1, under the conditions of normal pressure and a GHSV of 1,200 hr$^{-1}$, by changing the reaction bath temperature so as to maintain the 1-butene conversion ratio = 98.0 $\pm$ 1.0%. Thus, a coke-like substance was precipitated on the catalyst. A liquid component and a gas component were separated at the reaction tube outlet using a condenser, and the various components in the gas component were quantitatively analyzed using a gas chromatograph equipped with a hydrogen flame ionization detector and a thermal conductivity detector. The various data obtained by gas chromatography were compensated by multiplying by a factor, and thus the 1-butene conversion ratio and the butadiene yield were calculated. The butadiene yield for the TOS of 260 hours was 86.2%.

(Coke combustion reaction)

After the coke precipitation reaction, for the purpose of combusting the coke-like substance precipitated on the catalyst, a combustion reaction was performed for about a TOS of 10 hours at a reaction bath temperature of 400°C using a mixed gas having a gas volume ratio of oxygen : nitrogen = 1 : 3 at normal pressure and at a space velocity of 400 hr$^{-1}$. A quantitative analysis similar to that performed for the coke precipitation reaction was performed, and the amount of the coke-like substance was calculated from the cumulative amounts of production of $CO_2$ and CO in the reaction tube outlet gas, as a percentage with respect to the weight of the packed catalyst.

Test Example 2

The amount of the coke-like substance precipitated under the same reaction conditions as in Test Example 1, except that the TOS of the coke precipitation reaction was changed to 360 hours, was evaluated. The butadiene yield for a TOS of 360 hours was 86.2%.

Test Example 3

The amount of the coke-like substance precipitated under the same reaction conditions as in Test Example 1, except that the catalyst to be evaluated was changed to Catalyst 4, was evaluated.

Test Example 4

The amount of the coke-like substance precipitated under the same reaction conditions as in Test Example 1, except that the catalyst to be evaluated was changed to Catalyst 5, was evaluated.

Test Example 5

The amount of the coke-like substance precipitated under the same reaction conditions as in Test Example 1, except that the catalyst to be evaluated was changed to Catalyst 6, was evaluated.

Test Example 6

The amount of the coke-like substance precipitated under the same reaction conditions as in Test Example 1, except that the catalyst to be evaluated was changed to Catalyst 7, was evaluated.

Test Example 7

The amount of the coke-like substance precipitated under the same reaction conditions as in Test Example 1, except that the catalyst to be evaluated was changed to Catalyst 8, was evaluated.

Comparative Test Example 1

The amount of the coke-like substance precipitated under the same reaction conditions as in Test Example 1, except that the catalyst to be evaluated was changed to Catalyst 2 for comparison, was evaluated. The butadiene yield for a TOS of 260 hours was 87.5%.

Comparative Test Example 2

The amount of the coke-like substance precipitated under the same reaction conditions as in Comparative Test Example 1, except that the TOS for the coke precipitation reaction was changed to 360 hours, was evaluated. The butadiene yield for a TOS of 360 hours was 85.5%.

Comparative Test Example 3

The amount of the coke-like substance precipitated under the same reaction conditions as in Test Example 1, except that the catalyst to be evaluated was changed to Catalyst 3 for comparison, was evaluated. The butadiene yield for a TOS of 260 hours was 85.4%.

Comparative Test Example 4

The amount of the coke-like substance precipitated under the same reaction conditions as in Test Example 1, except that the catalyst to be evaluated was changed to Catalyst 9 for comparison, was evaluated.
The results for the coke precipitation reactions performed in Test Examples and Comparative Test Examples described above are presented in Table 1. As is obvious from Table 1, it is understood that in regard to the present catalysts, if the solid acidity is within the ranges defined in the present invention, the production of the coke-like substance may be suppressed to a large extent.

[Table 1]

| | Catalyst | Solid acidity | TOS for coke precipitation reaction | Amount of coke-like substance | Atomic ratio of Bi/ (AM + AEM) |
|---|---|---|---|---|---|
| | | [$\mu$mol/g] | [hr] | [wt%] | |
| Test Example 1 | Catalyst 1 | 98 | 290 | 0.01 | 6.0 |
| Test Example 2 | Catalyst 1 | 98 | 360 | 0.35 | 6.0 |
| Test Example 3 | Catalyst 4 | 68 | 290 | 0.13 | 1.4 |
| Test Example 4 | Catalyst 5 | 47 | 290 | 0.25 | 1.4 |
| Test Example 5 | Catalyst 6 | 49 | 290 | 0.05 | 10.0 |
| Test Example 6 | Catalyst 7 | 39 | 290 | 0.16 | 8.7 |
| Test Example 7 | Catalyst 8 | 42 | 290 | 0.15 | 10.0 |
| Comparative Test Example 1 | Catalyst 2 | 173 | 290 | 0.60 | 11.3 |
| Comparative Test Example 2 | Catalyst 2 | 173 | 360 | 1.89 | 11.3 |
| Comparative Test Example 3 | Catalyst 3 | 34 | 290 | 1.05 | 0.5 |
| Comparative Test Example 4 | Catalyst 9 | 30 | 290 | 0.40 | 0.5 |

**Claims**

1. A composite metal oxide catalyst for producing a conjugated diolefin by a gas phase catalytic oxidative dehydrogenation reaction from a mixed gas including a monoolefin having 4 or more carbon atoms and molecular oxygen, the composite metal oxide catalyst having a solid acidity of 35 to 172 $\mu$mol/g.

2. The composite metal oxide catalyst according to claim 1, comprising molybdenum (Mo), bismuth (Bi), an alkali metal (AM), and if necessary, an alkaline earth metal (AEM),
wherein a molar ratio of the alkali metal and the alkaline earth metal with respect to bismuth, Bi/(AM + AEM), is from 0.6 to 11.0.

3. The composite metal oxide catalyst according to claim 1 or 2, wherein the composite metal oxide catalyst satisfies the following composition formula:

$$Mo_{12}Bi_aFe_bCo_cNi_dX_eY_fZ_gO_h$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium and barium; Z represents at least one element selected from lanthanum, cerium, praseodymium, neodymium, samarium, europium, antimony, tungsten, lead, zinc, cerium and thallium; O represents oxygen; a, b, c, d, e, and f represent atomic ratios of bismuth, iron, cobalt, nickel, the alkali metal, the alkaline earth metal, and Z, respectively, with respect to molybdenum 12; and h represents a value that satisfies oxidation states of other elements in ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$.

4.  A method for producing the composite metal oxide catalyst for conjugated diolefin production according to claim 2, the method comprising steps of:

    preparing a slurry including a compound containing molybdenum; a compound containing bismuth; a compound containing at least one alkali metal; and if necessary, a compound containing at least one alkaline earth metal, and drying the slurry to obtain a dry powder; and
    calcining the dry powder at a temperature of from 200°C to 600°C.

5.  A method for producing the composite metal oxide catalyst for conjugated diolefin production according to claim 3, the method comprising steps of:

    preparing a slurry including a compound containing molybdenum; a compound containing bismuth; a compound containing iron; a compound containing cobalt; a compound containing nickel; a compound containing at least one alkali metal element selected from lithium, sodium, potassium, rubidium, and cesium; a compound containing at least one alkaline earth metal element selected from magnesium, calcium, strontium, and barium; and a compound containing at least one element Z selected from lanthanum, cerium, praseodymium, neodymium, samarium, europium, antimony, tungsten, lead, zinc, cerium, and thallium,
    provided that a, b, c, d, e, and f represent the atomic ratios of bismuth, iron, cobalt, nickel, the alkali metal, the alkaline earth metal, and Z, respectively, with respect to molybdenum 12, and in the ranges of $0.3 < a < 3.5$, $0.6 < b < 3.4$, $5 < c < 8$, $0 < d < 3$, $0 < e < 0.5$, $0 \leq f \leq 4.0$, $0 \leq g \leq 2.0$;
    drying the slurry to obtain a dry powder; and
    calcining the dry powder at a temperature of from 200°C to 600°C.

6.  A supported catalyst for producing conjugated diolefin, the supported catalyst having the composite metal oxide catalyst according to any one of claims 1 to 3 supported on a support.

7.  The supported catalyst according to claim 6, wherein the support is a spherical support.

8.  The supported catalyst according to claim 6 or 7, wherein the average particle size is from 3.0 mm to 10.0 mm, and the support ratio of the composite metal oxide catalyst is from 20% by weight to 80 weight.

9.  A method for producing the supported catalyst for conjugated diolefin production according to any one of claims 6 to 8, the method comprising a molding step of coating a support with a composite metal oxide catalyst together with a binder.

## INTERNATIONAL SEARCH REPORT

| International application No. |
| --- |
| PCT/JP2016/056414 |

A. CLASSIFICATION OF SUBJECT MATTER

*B01J23/887*(2006.01)i, *B01J35/08*(2006.01)i, *B01J37/02*(2006.01)i, *B01J37/08*(2006.01)i, *C07C5/48*(2006.01)i, *C07C11/167*(2006.01)i, *C07B61/00*(2006.01)n

According to International Patent Classification (IPC) or to both national classification and IPC

B. FIELDS SEARCHED

Minimum documentation searched (classification system followed by classification symbols)
B01J23/887, B01J35/08, B01J37/02, B01J37/08, C07C5/48, C07C11/167, C07B61/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2013-146655 A (Mitsubishi Chemical Corp.), 01 August 2013 (01.08.2013), claims 1 to 5; paragraphs [0051] to [0072] (Family: none) | 1-9 |
| A | JP 2014-198334 A (Mitsubishi Chemical Corp.), 23 October 2014 (23.10.2014), claims 1 to 5; paragraphs [0053] to [0069] (Family: none) | 1-9 |
| A | JP 2011-177616 A (Nippon Shokubai Co., Ltd.), 15 September 2011 (15.09.2011), claims 1 to 4; paragraphs [0035] to [0037] (Family: none) | 1-9 |

| ☒ Further documents are listed in the continuation of Box C. | ☐ See patent family annex. |
| --- | --- |

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 24 March 2016 (24.03.16) | 05 April 2016 (05.04.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

International application No.

PCT/JP2016/056414

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-100244 A (Mitsui Chemicals, Inc.), 23 May 2013 (23.05.2013), claims 1 to 7 (Family: none) | 1-9 |
| A | JP 63-077827 A (Keishitsu Ryubun Shin Yoto Kaihatsu Gijutsu Kenkyu Kumiai), 08 April 1988 (08.04.1988), claims 1 to 16 (Family: none) | 1-9 |
| A | WO 2014/061711 A1 (Asahi Kasei Chemicals Corp.), 24 April 2014 (24.04.2014), claims 1 to 6; paragraph [0063] & US 2015/0210614 A1 claims 9 to 21; paragraph [0074] & EP 2910539 A1 | 1-9 |

Form PCT/ISA/210 (continuation of second sheet) (January 2015)

**REFERENCES CITED IN THE DESCRIPTION**

**Patent documents cited in the description**

- JP 2013100244 A
- JP 2011148765 A
- JP 2013146655 A
- JP 2013202459 A